# EUROPEAN PATENT SPECIFICATION

(11) **EP 0 299 758 B1**
(45) Date of publication and mention of the grant of the patent: **14.12.1994**
(21) Application number: 88306442.0
(22) Date of filing: 14.07.1988
(51) Int. Cl.: C07C 271/06, C08G 18/48, A61K 47/02

(54) **Compositions for enhancing the cutaneous penetration of pharmacologically active agents**
Mittel zur Steigerung der Hautpenetration von pharmakologisch aktiven Verbindungen
Compositions augmentant la pénétration cutanée d'agents pharmaceutiquement actifs

(30) Priority: 16.07.1987 US 74262; 08.07.1988 US 216804
(43) Date of publication of application: 18.01.1989
(73) Proprietor: THE REGENTS OF THE UNIVERSITY OF CALIFORNIA, Berkeley, California 94720 (US)
(72) Inventor: Chess, Samuel, Newport Beach California 92660 (US); McCullough, Jerry L., Irvine California 92714 (US); Weinstein, Gerald D., Irvine California 92715 (US)
(74) Representative: Goldin, Douglas Michael

(56) References cited:
- FR-A- 2 557 576
- GB-A- 1 179 231
- US-A- 2 814 605
- US-A- 3 477 977
- US-A- 3 539 482
- US-A- 3 822 238
- US-A- 4 079 028
- US-A- 4 543 405

## Description

This invention relates generally to topical or transdermal administration of pharmacologically active agents and more particularly relates to methods and compositions for enhancing the permeability of the skin to achieve enhanced cutaneous penetration of such agents.

The delivery of drugs through skin provides many advantages; primarily, such a means of delivery is a comfortable, convenient and non-invasive way of administering drugs. The variable rates of absorption and metabolism encountered in oral treatment are avoided, and other inherent inconveniences -- e.g., gastrointestinal irritation and the like -- are eliminated as well. Transdermal drug delivery also makes possible a high degree of control over blood concentrations of any particular drug.

Skin is a structurally complex, relatively thick membrane. Molecules moving from the environment into and through intact skin must first penetrate the stratum corneum and any material on its surface. They must then penetrate the viable epidermis, the papillary dermis, and the capillary walls into the bloodstream or lymph channels. To be so absorbed, molecules must overcome a different resistance to penetration in each type of tissue. Transport across the skin membrane is thus a complex phenomenon. However, it is the cells of the stratum corneum which present the primary barrier to absorption of topical compositions or transdermally administered drugs. The stratum corneum is a thin layer of dense, highly keratinized cells approximately 10-15 microns thick. It is believed to be the high degree of keratinization of these cells which creates in most cases a substantially impermeable barrier to drug penetration.

In order to increase skin permeability, and in particular to increase the permeability of the stratum corneum, the skin may be pretreated with one or more penetration-enhancing agents prior to application of a medicament. Alternatively, and preferably, a drug and potentiator are concurrently delivered.

Various compounds for enhancing the permeability of skin are known in the art. U.S. Patent Nos. 4,006,218, 3,551,554 and 3,472,931, for example, respectively describe the use of dimethylsulfoxide (DMSO), dimethyl formamide (DMF) and N,N-dimethylacetamide (DMA) to enhance the absorption of topically applied drugs through the stratum corneum. Other compounds which have been used to enhance skin permeability include decylmethylsulfoxide (C₁₀MSO), polyethylene glycol monolaurate (PEGML; see, e.g., U.S. Patent No. 4,568,343), and the 1-substituted azacycloheptan-2-ones, particularly 1-n-dodecylcyclazacycloheptan-2-one (available under the trademark "Azone" from Nelson Research & Development Co., Irvine, CA; see U.S. Patent Nos. 3,989,816, 4,316,893 and 4,405,616). The compounds of the prior art, however, suffer from one or more disadvantages. Clinical use has been limited by either the penetration of the agent itself, a problem widely noted with DMSO, or by cutaneous irritation, encountered, inter alia, with C₁₀MSO and the 1-substituted azacycloheptan-2-ones.

In selecting a penetration-enhancing potentiator, or "permeation enhancer," it is essential that adverse systemic effects and irreversible damage to the skin structure be avoided. It is also desirable that the compound itself not cause irritation or allergic response.

### Disclosure of Invention

Accordingly, it is an object of the present invention to overcome the aforementioned disadvantages of the prior art.

It is another object of the invention to provide a composition for topical or transdermal administration of a pharmacologically active agent which increases the permeability of skin or mucosa and thus allows for enhanced cutaneous penetration of the agent. In addition to a selected pharmacologically active agent, the composition contains one or more urethane compounds as a permeation enhancer, preferably dispersed within a topical carrier, and a selected pharmacologically active agent.

It is still another object of the invention to provide a method of increasing the permeability of the skin and thus enhancing the cutaneous penetration of a pharmacologically active agent, the method comprising applying the agent to skin or other epithelial tissue in conjunction with one or more urethane compounds that serves as a permeation enhancer.

It has now been discovered that nonpolymeric hydroxy- or alkoxy-terminated urethane compounds which may be prepared by reacting two moles of a hydroxy- or hydroxy/alkoxy-terminated linear polyalkylene glycol or polyether with one mole of a monomeric organic diisocyanate are excellent permeation enhancers which potentiate or facilitate the skin's permeability to pharmacologically active agents with which these discrete urethane compounds are formulated.

The permeation enhancing compounds used according to the invention have the formula: 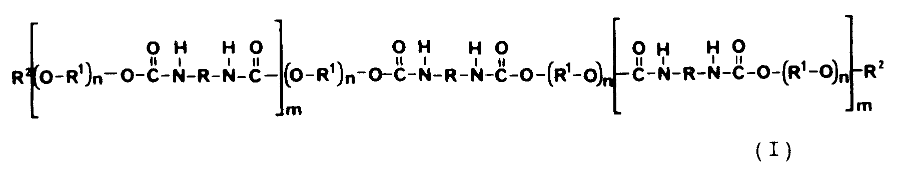
wherein R is an alkylene or alkenylene radical containing from one to 20 carbon atoms, or a cycloalkylene or cycloalkylene radical containing from 5 to 10 carbon atoms, or a mononuclear or fused ring arylene radical containing from 6 to 10 carbon atoms (which R radicals may be unsubstituted or substituted);
R¹ is the same or different alkylene or alkenylene radical; R² is hydrogen or a methyl or ethyl group; m is a number in the range of 0 and 6 inclusive, more preferably 0 and 3 inclusive; and n is selected to provide the enhancer with a molecular weight of at least 200. Thus n is such that each polyalkylene glycol or polyether moiety (R¹-O)ₙ has a molecular weight in the range of from about 60 to 6000, more typically 100 to 1000; these values giving the enhancer compound itself a molecular weight of at least 200, more typically 200 to 37,000, still more typically 250 to 2200.

According to one aspect of the invention, a composition is provided for achieving enhanced penetration of a pharmacologically active agent applied dermally or to other epithelial tissue, comprising:
(a) a therapeutically effective amount of a pharmacologically active agent; and
(b) an amount of a permeation enhancing compound of formula I as described above effective to enhance the penetration of the pharmacologically active agent.

The compositions according to the invention may be used administering a drug comprising contacting skin or other epithelial tissue, e.g. mucosal tissue, with a composition containing one or more of the aforementioned urethane compounds as a permeation enhancer and a selected pharmacologically active agent.

In another embodiment, the invention provides a compound of the formula I for use in medical treatment by enhancing the penetration of a pharmacologically active agent applied dermally or to other epithelial tissue.

### Brief Description of Drawings

Figure 1 graphically illustrates a comparison of penetration enhancement provided by methotrexate compositions prepared according to the invention with that provided by an optimized standard;
Figure 2 graphically illustrates a comparison of penetration enhancement provided by enhancer compositions prepared according to the invention with that provided by a known composition; and
Figure 3 graphically illustrates a comparison of penetration enhancement provided by urea compositions prepared according to the invention with that provided by urea compositions formulated with a known enhancer.

### Modes for Carrying Out the Invention

The invention relates primarily to compositions for topical or transdermal administration of a pharmacologically active agent, which compositions increase the permeability of the skin or other epithelial tissue and thus allow for enhanced cutaneous penetration. The compositions of the invention include one or more nonpolymeric hydroxy- or alkoxy-terminated urethane compounds as a permeation enhancer, preferably dispersed within a suitable topical carrier, and a selected pharmacologically active agent.

The aforementioned nonpolymeric hydroxy- or alkoxy-terminated urethane compounds are represented by the general formula: 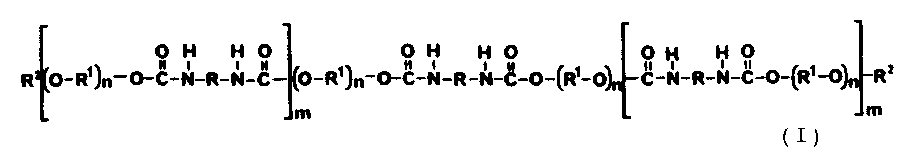
wherein R represents an alkylene or alkenylene radical containing from one to 20 carbon atoms, such as methylene, trimethylene and dimethyltrimethylene radicals, and in the case of the alkenylene radicals, one having between one and 3 double bonds, or a cycloalkylene or cycloalkenylene radical, containing from 5 to 10 carbon atoms, such as cyclopentylene, cyclohexylene and cyclohexenylene radicals, or a mononuclear or fused ring arylene radical, containing from 6 to 10 carbon atoms, such as phenylene or naphthylene, all of which can be unsubstituted or substituted, e.g., with alkyl groups, generally ones containing up to about 6 carbon atoms, aryl groups which may be substituted with amine moieties, nitro, lower (1-6C) alkyl, lower (1-6C) alkoxy, lower (1-6C) alkylether, halogen, and the like. R¹ represents the same or different alkylene or alkenylene radicals, generally ones containing from 2 to 6 carbon atoms, such as -CH₂CH₂- and -CH₂CH₂CH₂-, and in the case of the alkenylene radicals, ones typically having one or two double bonds; R² is hydrogen or a methyl or ethyl group, and m is a number in the range of 0 and 6 inclusive, more preferably 0 and 3 inclusive, and n is an integer selected to provide each polyalkylene glycol or polyether moiety (R¹-O)ₙ with a molecular weight in the range of from about 60 to 6000, more typically 100 to 1000. These values give the enhancer compound itself a molecular weight of at least 200, typically 200 to 37,000, more typically 250 to 2200.

"Penetration enhancement" or "permeation enhancement" as used herein relates to increasing the permeability of skin to one or more pharmacologically active agents so as to allow for cutaneous delivery of a pharmacologically active agent. The enhanced penetration effected through the use of the inventive compositions can be observed, for example, by measuring the rate of diffusion of pharmacologically active agent through animal or human skin using a diffusion cell apparatus.

By "cutaneous" or "dermal" delivery, applicants intend to include both transdermal (or "percutaneous") administration, i.e. delivery by actual passage of a pharmacologically active agent through the skin (or other epithelial tissue such as buccal mucosa) into the blood stream, and local administration of a topical pharmacologically active agent as in, for example, the treatment of various skin disorders.

"Topical carriers" as used herein refer to carrier materials suitable for topical applications of drugs or cosmetics, and include any such materials known in the cosmetic and medical arts, e.g., any liquid, gel, solvent, liquid diluent, or the like, which does not adversely affect living animal tissue or interact with other components of the composition in a deleterious manner. Topical carriers are used to provide the compositions of the invention in their preferred liquid form. Examples of suitable topical carriers for use herein include water, liquid alcohols, liquid glycols, liquid polyalkylene glycols, liquid esters, liquid amides, liquid protein hydrolysates, liquid alkylated protein hydrolysates, liquid lanolin and lanolin derivatives, and like materials.

By the term "pharmacologically active agent" or "drug" as used herein is meant any chemical material or compound suitable for topical or transdermal administration which induces any desired local or systemic effect. Such substances include, the broad classes of compounds normally delivered through body surfaces and membranes, including skin. In general, this includes therapeutic agents in all of the major therapeutic areas including, but not limited to, anti-infectives such as antibiotics and antiviral agents, analgesics and analgesic combinations, anorexics, anthemidines, antiarthritics, antiasthmatic agents, anticonvulsants, antidepressants, antidiabetic agents, antidiarrheals, antihistamines, antiinflammatory agents, antimigraine preparations, antimotion sickness, antinauseants, antineoplastics, antiparkinsonism drugs, antipruritics, antipsychotics, antipyretics, antispasmodics, including gastrointestinal and urinary, anticholinergics, sympathomimetics, xanthine derivatives, cardiovascular preparations including calcium channel blockers, beta-blockers, antiarrhythmics, antihypertensives, diuretics, vasodilators including general coronary, peripheral and cerebral, central nervous system stimulants, cough and cold preparations, decongestants, diagnostics, hormones, hypnotics, immunosuppressives, muscle relaxants, parasympatholytics, parasympathomimetics, psychostimulants, sedatives and tranquilizers.

Locally administered topical medicaments also fall within this category and include, for example, antibiotics, antifungals, antimicrobials, cutaneous growth enhancers including for the hair and nails, pigment modulators, antiproliferatives, antipsoriatics, retinoids, anti-acne medicaments, antineoplastic agents, phototherapeutic agents, sunscreens, cutaneous protection agents, alpha-hydroxy acids including lactic and glycolic acids, and the like.

By "effective" amount of a pharmacologically active agent is meant a nontoxic but sufficient amount of a compound to provide the desired local or systemic effect and performance at a reasonable benefit/risk ratio attending any medical treatment. An "effective" amount of a permeation enhancer as used herein means an amount selected so as to provide the desired increase in skin permeability and, correspondingly, the desired depth of penetration, the rate of administration, and the amount of drug delivered.

The hydroxy- or hydroxy/alkoxy-terminated linear polyalkylene glycols or polyethers reacted with monomeric organic diisocyanates to form the nonpolymeric hydroxy- or alkoxy-terminated urethane penetration enhancers of this invention are represented by the general formula:

R²(O-R¹)ₙ-O-R² (II)

wherein R¹, and R² and n are as defined hereinabove for formula I. Included among these polyalkylene glycols or polyethers are polyalkylene ether glycols, such as polyethylene glycols, polypropylene glycols, polybutylene glycols, polytetramethylene glycols, polyhexamethylene glycols, polypropenylene glycols, and the like, which are obtained, for example, by acid-catalyzed condensation of lower alkylene oxides, such as ethylene oxide, propylene oxide, and the like, either with themselves or with glycols such as ethylene glycol, propylene glycol, propenylene glycol, and the like.

Polyalkylenearylene ether glycols which also have molecular weights ranging from about 60 to about 6000, more typically 100 to 1000, but which differ from the above-described polyalkylene glycols in having cycloalkylene or cycloalkenylene radicals, generally ones which contain from 5 to 10 carbon atoms, such as cyclopentylene, cyclohexylene, and cyclohexenylene radicals, or mononuclear or fused ring arylene radicals, all of which may either be unsubstituted or substituted, e.g., with alkyl groups, generally ones containing up to 6 carbon atoms, amines, nitro, lower alkoxy, lower alkylether, halogen and the like, in place of some of the alkylene or alkenylene radicals of said polyalkylene glycols, may also be employed as polyalkylene glycol or polyether reactants.

Specific polyalkylene glycol or polyether reactants coming within the scope of formula II hereinabove include:
diethylene glycol,
triethylene glycol,
polyethylene glycol 300,
polyethylene glycol 400,
polyethylene glycol 600,
polyethylene glycol 900,
polyethylene glycol 1000,
polyethylene glycol 2000,
polypropylene glycol 400,
polypropylene glycol 700,
polypropylene glycol 1000,
polypropylene glycol 1200,
polypropylene glycol 2000,
polypropylene glycol 3000,
polypropylene glycol 4000,
polypropylene glycol 6000,
ethylene oxide end-capped polypropylene glycol 1000,
ethylene oxide end-capped polypropylene glycol 2000,
ethylene oxide end-capped polypropylene glycol 3000,
ethylene oxide end-capped polypropylene glycol 4000,
and the like.

As can readily be appreciated, mixtures of the various reactive organic polyalkylene glycols or polyethers described hereinabove may also be employed in preparing the urethane penetration-enhancers used in the practice of the present invention.

A wide variety of monomeric organic diisocyanates represented by the general formula:

O=C=N-R-N=C-O (III)

wherein R is as defined hereinabove for formula I, can be used to form the nonpolymeric hydroxy- or alkoxy-terminated urethane penetration enhancers of this invention. Included among such diisocyanates are aromatic diisocyanates, such as m-phenylenediisocyanate, p-phenylenediisocyanate, 4-t-butyl-m-phenylenediisocyanate, 4-methoxy-m-phenylenediisocyanate, 4-phenoxy-m-phenylenediisocyanate, 4-chloro-m-phenylenediisocyanate, toluenediisocyanates (either as a mixture of isomers, e.g., the commercially available mixture of 80% 2,4-toluenediisocyanate and 20% 2,6-toluenediisocyanate, or as the individual isomers themselves), m-xylylenediisocyanate, p-xylylenediisocyanate, cumene-2,4-diisocyanate, durenediisocyanate, 1,4-naphthylenediisocyanate, 1,5-naphthylenediisocyanate, 1,8-naphthylenediisocyanate, 2,6-naphthylenediisocyanate, 1,5-tetrahydronaphthylenediisocyanate, p,p'-diphenyldiisocyanate, diphenylmethane-4,4'-diisocyanate, 2,4-diphenylhexane-1,6-diisocyanate, "bitolylenediisocyanate" (3,3'-dimethyl-4,4'-biphenylenediisocyanate), "dianisidinediisocyanate" (3,3'-dimethoxy-4,4'-biphenylenediisocyanate); aliphatic diisocyanates, such as methylenediisocyanates, ethylenediisocyanate, the tri-, tetra-, penta-, hexa-, octa-, nona- and decamethylene-ω,ω-diisocyanates, 2-chloro-trimethylenediisocyanate, 2,3-dimethyltetramethylenediisocyante, and the like, as well as mixtures thereof.

The urethanes of formula I above may be used to pretreat skin or other epithelial tissue prior to topical or transdermal administration of a pharmacologically active agent. Alternatively, and preferably, the penetration-enhancing urethane is administered concurrently with the pharmacologically active agent. The potentiator compound and drug may be administered with or without a topical carrier, although such a carrier is preferred. Where the composition includes a topical carrier, a formulation comprises from 5-90 wt% urethane compound, usually from 5-25 wt% urethane compound, and typically up to about 75 wt%, carrier. Exemplary carriers herein include water, alcohols, including both monohydric and polyhydric alcohols, e.g. ethanol, isopropanol, glycerol, sorbitol, 2-methoxyethanol, diethylene glycol, ethylene glycol, hexylene glycol, mannitol and propylene glycol; ethers such as diethyl or dipropyl ether; polyethylene glycols and methoxypolyoxyethylenes; carbowaxes having molecular weights ranging from 200 to 20,000; polyoxyethylene glycerols; polyoxyethylene; sorbitols; and stearoyl diacetin. The topical carrier preferably includes both alcohol and water so as to accommodate lipophilic and hydrophilic drugs. An example of a particularly preferred formulation is a composition containing 25 wt% urethane compound and 75 wt% topical carrier, the carrier comprising 60 wt% isopropanol or ethanol and 40 wt% water.

The topical carriers described herein include various agents and ingredients commonly employed in dermatological and cosmetic ointments and lotions. For example, excipients, fragrances, opacifiers, preservatives, anti-oxidants, gelling agents, perfumes, thickening agents such as carboxymethylcellulose, stabilizers, surfactants, emollients, coloring agents and the like may be present.

It should be appreciated that the composition of the carrier can be varied to complement of the structure of the drug by making it more hydrophilic, e.g. by addition of water, or by making it more lipophilic, e.g. by addition of a long-chain alcohol.

The amount of pharmacologically active agent similarly will depend on a variety of factors, including the disease to be treated, the nature and activity of the agent, the desired effect (systemic or local or both), possible adverse reactions, the ability and speed of the agent selected to reach its intended target, the cost and availability of the agent, the use of two or more pharmacologically active agents, and other factors within the particular knowledge of the patient and physicians.

As noted above, the compositions of the present invention may be used with any number of pharmacologically active agents which may be topically or transdermally administered. Examples of exemplary pharmacologically active agents suitable for use in conjunction with the present invention include urea, vasodilators such as minoxidil, and lactic acid, e.g. in treating X-linked ichthyosis, a genetic skin disease characterized by excessive scaling.

Any particular formulation will further depend on the chemical nature of the pharmacologically active agent to be administered as well as on the depth of cutaneous or mucosal penetration desired. The depth of penetration varies with the structure and molecular weight of the urethane compound chosen as a permeation enhancer. The method of delivery may also vary, but necessarily involves applying the formulation of the present invention to skin or other epithelial tissue for a period of time sufficient to allow the desired penetration of the selected drug or topical medicament. The method may involve direct application of the inventive composition as an ointment, gel, cream, or the like, or may involve use of a drug delivery device as taught, for example, in U.S. Patent Nos. 3,742,951, 3,797,494 or 4,568,343.

Preferred drug delivery devices include a drug/permeation enhancer reservoir in the form of a matrix comprising rubber or other polymeric material, e.g. natural and synthetic rubbers such as polybutylene, polyisobutylene, polybutadiene, polyethylene, styrenebutadiene copolymers, polyisoprene, polyurethane, ethylene/propylene copolymers, polyalkylacrylate polymers, copolyesters, ethylene/acrylic copolymers, silicones and butadiene/acrylonitrile copolymers, ethylene vinylacetate, gelled or thickened mineral oil, petroleum jelly and various aqueous gels and hydrophilic polymers. The matrix is applied to skin using a suitable adhesive as described, for example, in U.S. Patent No. 4,568,343, supra.

Methods of synthesizing urethanes have long been known in the art. U.S. Patent No. 2,266,777, for example, issued in 1941, describes the reaction of isocyanate compounds with polyhydroxy alcohols to give polyurethanes, now a standard synthetic method. Reference may also be had to Saunders, Polyurethanes: Chemistry and Technology (New York: Wiley & Sons, 1961) for an overview of the chemistry of urethanes.

In preparing the urethane compounds of formula I, wherein R² is hydrogen, the mole ratio of diol to diisocyanate is about 2:1, and the reaction is carried out at elevated temperature (at least 37.8^{o}C (100^{o}F) and preferably at least 65.6^{o}C (150^{o}F); temperatures may be higher depending on the particular reactants and on the amount of catalyst used) with constant mixing. The solvent used is a suitable hydrocarbon solvent such as dioxane, xylene, cyclohexane or the like. The use of catalysts is optional. Suitable catalysts include organic tin alkyl titinates or octoates and amines such as those in the Dabco group (i.e., Dabco DC-1, DC-2, R-8020, R-595, 33 LV, DF and WT, all available from Air Products and Chemicals, Inc., Allentown, PA, under the "Dabco" trademark) and N-ethyl morpholine. Further information regarding analogous reactions may be found in U.S. Pat. Nos. 2,266,777 and 2,282,827, the disclosures of which are hereby incorporated by reference in their entirety.

The urethane compounds of formula I, wherein R² is a methyl or ethyl group, are prepared by a similar method, i.e., using substantially the same reaction parameters: reacting a diisocyanate with a diol that has been partially capped with ethylene oxide or other suitable capping agent to give a terminal primary monohydroxy product. In this case, the mole ratio of capped diol to diisocyanate is also about 1:2.

The "m values" of the compounds of Formula I may be controlled during synthesis by varying the reaction temperature, the amount, if any, of water in the reaction mixture, and the starting materials. For example, a higher temperature will typically result in a more highly polymerized structure, i.e., one having a higher m value, while increasing the amount of water present will ordinarily give lower molecular weight compounds. Selection of reaction conditions herein is believed to be well within the skill of the art, and may in any case be readily derived from the aforementioned references on urethane chemistry.

### Example 1

Forty grams of polyethylene glycol 400 were admixed with 2-3 drops of stannous octoate in a 100 ml beaker, using a Teflon stirring bar, for 5 minutes. Then, 8.7 grams of toluene diisocyanate were slowly added, with stirring, in small increments, taking care not to let the reactions temperature exceed about 71.1^{o}C (160^{o}F). Once all the toluene diisocyanate had been added, the reaction mixture was stirred for an additional 40 minutes while the temperature was maintained at about 71.1^{o}C (160^{o}F), then allowed to cool to room temperature. The resulting urethane compound can be represented by the formula: 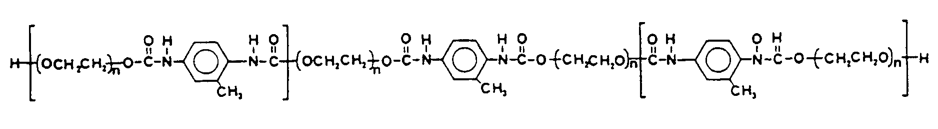
wherein n is a number such that the number of oxyethylene groups in each polyethylene glycol moiety give that moiety a molecular weight of about 500. In this example, as well as in the examples which follow, completion of the reaction and identification of the product were confirmed by infrared spectroscopy. Unless otherwise noted all reagents used in these examples were obtained from either Union Carbide Corporation (New York, NY) or Dow Chemical (Midland, MI).

### Example II

Forty grams of polyethylene glycol 400 were admixed with 0.08 grams of stannous octoate in a 100 ml beaker, using a Teflon stirring bar, for 5 minutes. Then, 13.1 grams of dicyclohexylmethane diisocyanate were slowly added, with stirring. After all of the dicyclohexylmethane diisocyanate was added, the mixture was stirred for an additional 30 minutes. Then, an additional 1 cc of stannous octoate was added, and the mixture was stirred for an additional 10 minutes. At this time, an exothermic effect was noted. The mixture was allowed to react at 71.1^{o}C (160^{o}F) for 2 hours, then cooled to room temperature.

### Examples III - XI

A series of urethane compounds were prepared using different polypropylene glycols listed in Table I and dicyclohexylmethane diisocyanate. The procedure of Example I was followed, using two moles of polypropylene glycol to each mole of dicyclohexylmethane diisocyanate, except that the reaction mixture was maintained at a temperature between 65.6^{o}C and 71.1^{o}C (between 150^{o}F and 160^{o}F) while being stirred for an additional 30 minutes.

**Table I**

| Example | Polypropylene Glycol |
|---|---|
| III | Dipropylene Glycol |
| IV | Polypropylene Glycol 410 |
| V | Polypropylene Glycol 701 |
| VI | Polypropylene Glycol 1000 |
| VII | Polypropylene Glycol 1200 |
| VIII | Polypropylene Glycol 2000 |
| IX | Polypropylene Glycol 2000 |
| X | Polypropylene Glycol 3000 |
| XI | Polypropylene Glycol 4010 |

### Examples XII - XVIII

The procedure of Example I was followed to prepare a series of urethane compounds using the different polypropylene glycols listed in Table II and toluene diisocyanate, in the ratio of two moles of polypropylene glycol to one mole or toluene diisocyanate.

**Table II**

| Example | Polypropylene Glycol |
|---|---|
| XII | Dipropylene Glycol |
| XIII | Polypropylene Glycol 400 |
| XIV | Polypropylene Glycol 700 |
| XV | Polypropylene Glycol 1000 |
| XVI | Polypropylene Glycol 2000 |
| XVII | Polypropylene Glycol 3000 |
| XVIII | Polypropylene Glycol 4000 |
| X | Polypropylene Glycol 3000 |
| XI | Polypropylene Glycol 4010 |

### Examples XIX - XXI

A series of urethane compounds were prepared using different polypropylene glycols, listed in Table III, and methylene bis(4-phenylisocyanate). The procedures and conditions of Example I were followed with the exception that the reaction mixture was heated to, and maintained at, a temperature between 71.1^{o}C and 76.7^{o}C (between 160^{o}F and 170^{o}F) while being stirred for an additional hour.

**Table III**

| Example | Polypropylene Glycol |
|---|---|
| XIX | Polypropylene Glycol 1000 |
| XX | Polypropylene Glycol 1200 |
| XXI | Polypropylene Glycol 2000 |

### Example XXII

Using the procedure and reaction conditions of Example I, 72.5 grams of melted polyethylene glycol 1450 were admixed with 26.2 grams of dicyclohexylmethane diisocyanate and two drops of stannous octoate.

### Example XXIII

The procedure of Example III was repeated replacing the dipropylene glycol with 45 grams of polyethylene glycol 900.

### Example XXIV

Twenty grams of polypropylene glycol, m.w. approximately 2000 (manufactured by Olin Chemical Co., Stamford, CT, under the trademark "Poly G-55-56") were admixed with 1.25 grams of methylene bis(4-phenylisocyanate) and two drops of stannous octoate using the reaction conditions and procedure of Example XIX.

### Example XXV

The procedure of Example XXIV was repeated using 30 grams of polyethylene glycol 600, 6.55 grams of dicyclohexylmethane diisocyanate and two drops of stannous octoate.

### Example XXVI

The procedure of Example XXV was repeated using 30 grams of polyethylene glycol 600, 6.35 grams of 4,4-diphenylmethane diisocyanate, and three drops of stannous octoate.

### Example XXVII

The procedure of Example XIX was repeated using two moles of the different polypropylene glycols shown in Table IV and one mole of 4,4'-diphenylmethane diisocyanate.

**Table IV**

| Example | Polypropylene Glycol |
|---|---|
| LI | Polypropylene Glycol 3000 |
| LII | Polypropylene Glycol 4000 |
| LIII | Mixture of Polypropylene Glycol 1025 and 425--average mw = 700 |

### Example XXVIII

The procedure of Example XIX was repeated using 50 grams of polypropylene glycol 1025, four grams p-phenylene diisocyanate and three drops of stannous octoate, except the mixture was stirred for one hour at 60^{o}C to 65.6^{o}C (140^{o}F to 150^{o}F).

### Example XXIX

Thirty-six grams of melted polyethylene glycol 900 at 90^{o}F were admixed with 1.74 grams of toluene diisocyanate, using the reaction conditions and procedure of Example XX. The resulting urethane compound can be represented by the formula: 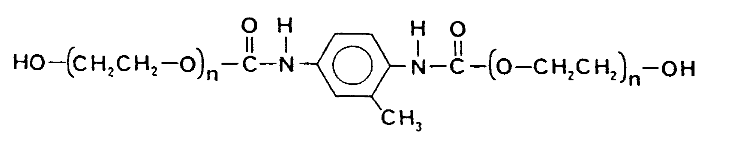
wherein n is a number such that the number of oxyethylene groups in each polyethylene glycol moiety give that moiety a molecular weight of about 900.

### Example XXX

Fifty grams of polypropylene glycol 1000 were admixed with 33.5 grams of polyethylene glycol 3350, 7.8 grams of dicyclohexylmethane diisocyanate and three drops of stannous octoate using the reaction conditions and procedures of Example XIX.

### Example XXXI

### In Vitro Percutaneous Penetration Studies

The urethane compounds of the preceding examples were used as vehicles for selected drugs (urea, hydrocortisone, methotrexate). In vitro skin transport studies were done to determine the effect of the urethane-containing vehicles on drug penetration. These tests were conducted using standard Franz in vitro percutaneous diffusion cells with excised pig skin (as described in T.J. Franz, J. Invest. Dermatol. 64:190-195 (1975) and in T.J. Franz, "The Finite Dose Technique as a Valid In Vitro Model for the Study of Percutaneous Absorption in Man" in Current Problems in Dermatology, vol. 7, pages 58-68, Ed. J.W.H. Mali (Karger, Basel 1978)).

Excised pig skin was mounted between the cylindrical glass cell cap and the cell body, with the epidermal surface facing outward, and the dermal surface bathed from below with isotonic saline. The temperature of the chamber was maintained at 37^{o}C by water circulated through the jacketed cell chamber. A Teflon-covered magnet stirring bar, driven by an external magnet mounted on a timing motor, kept the saline solution in constant motion. Radiolabeled drug was added to the test formulation as a tracer, which was uniformly applied in small quantities (0.05-0.25 grams total formulation) to the epidermal surface of the skin. At sequential time intervals (e.g., 6 hr, 12 hr, 36 hr, 48 hr) 1 ml aliquots of saline from the dermal reservoir were removed through the sampling port provided for that purpose. The radio-activity (tritum) or carbon-14 (¹⁴C) of the tracer compound which penetrated through the skin into the reservoir was determined by liquid scintillation counting. The total quantity of drug which penetrated for each time interval was then calculated based on the specific activity of the formulation which was applied to the skin's surface.

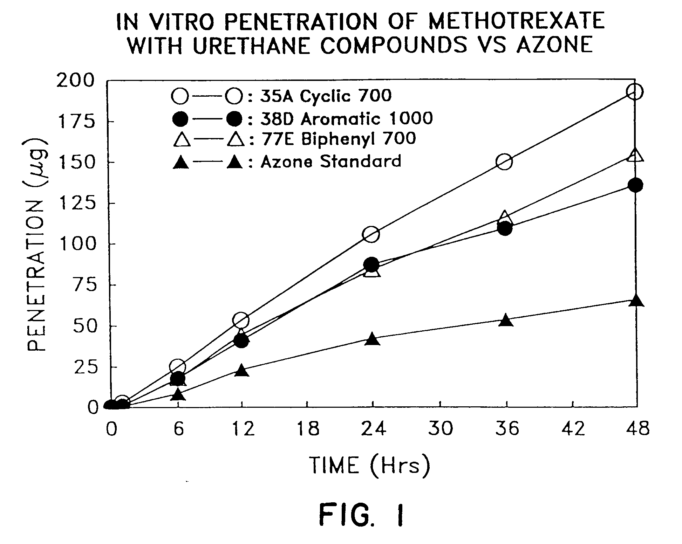

For these studies the urethane compounds were formulated into test vehicles by mixing 2.5 grams of urethane compound with 5.0 grams of isopropyl alcohol and 2.5 grams of distilled water. Tracer amounts of radiolabeled urea, hydrocortisone, or methotrexate were added to the vehicle, usually at a total drug concentration of 1%. The studies evaluated vehicles containing various urethane compounds, with diverse chemical structures and properties and varying molecular weights for their ability to facilitate drug transport across skin. Figures 1, 2 and 3 summarize the effects of selected urethane compounds in enhancing drug penetration through skin:
(a) Figure 1 shows that all urethane compounds tested produced enhanced percutaneous penetration of 1% methotrexate versus an optimized standard 1% methotrexate formulation. The compounds tested in Figure 1 were as follows: "35A Cyclic 700" was the reaction product of 2 moles polypropylene glycol, m.w. approximately 700, with 1 mole of dicyclohexylmethane diisocyanate; "38D Aromatic 1000" was the reaction product of 2 moles polypropylene glycol, m.w. approximately 1000, with 1 mole toluene diisocyanate; and "77E Biphenyl 700" was the reaction product of 2 moles of polypropylene glycol, m.w. approximately 700, with 1 mole diphenylmethane-4,4'-diisocyanate.

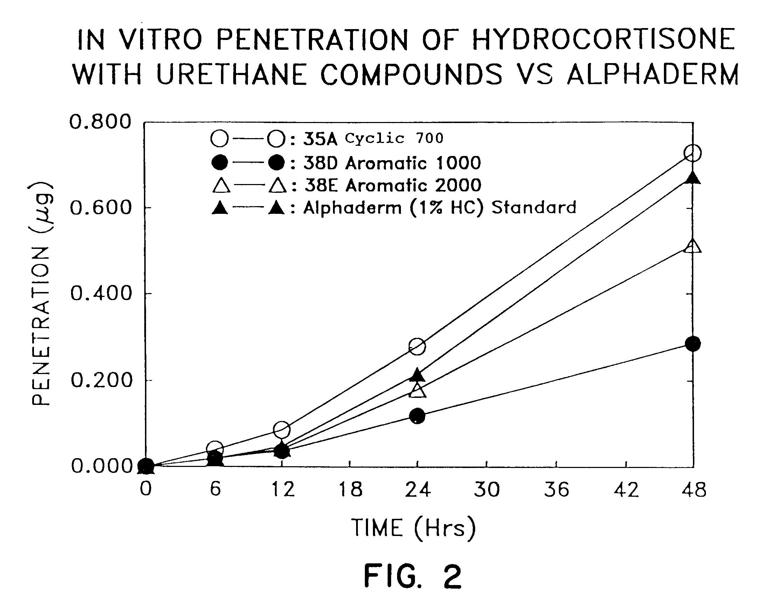

(b)In Figure 2, various aromatic urethane compounds with propylene glycol (PPG), molecular weight ranging from 700 to 2000 were compared with an optimized formulation of 1% hydrocortisone (available under the trademark "Alphaderm" from Morton-Norwich Products, Norwich, NY). (In Figure 2, compound "38E Aromatic 2000" was the reaction product of 2 moles polypropylene glycol, m.w. approximately 2000, with 1 mole of toluene diisocyanate.) The penetration of hydrocortisone in urethane compound 35A Cyclic 700 (see (a) above) was comparable to that of Alphaderm.

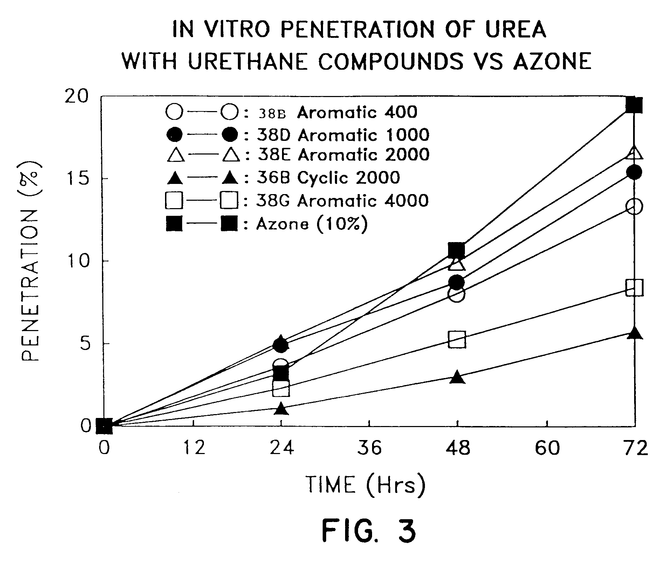

(c) In Figure 3, the penetration of 1% urea in various aromatic and cyclic urethane compounds with PPG molecular weight ranging from 400 to 4000 was compared with an Azone-containing formulation. The compounds used were as follows: "35B Aromatic 400" was the reaction product of 2 moles of polypropylene glycol, m.w. approximately 400, with 1 mole of toluene diisocyanate; "36B Cyclic 2000" was the reaction product of 2 moles of polypropylene glycol, m.w. approximately 2000, with 1 mole of dicyclohexylmethane diisocyanate; "38G Aromatic 4000" was the reaction product of 2 moles of polypropylene glycol, m.w. approximately 4000, with 1 mole polypropylene glycol. Maximum urea penetration was achieved with the Azone formulation, followed closely by the aromatic urethane compounds (38D/38E), formulated with polypropylene glycol, m.w. 1000-2000. The lowest urea penetration was obtained with the Cyclic 2000 and Aromatic 4000 urethane compounds.

### In Vivo Pharmacological Effects of Topical Drugs Formulated in Urethane Compounds

### Example XXXII

Topical application daily for 10 days of 1% methotrexate (MTX) in urethane compound 35A to the backs of hairless mice produced 47% inhibition of DNA synthesis in the treated skin, as assessed autoradiographically by epidermal labeling indices. The topically applied methotrexate was absorbed and produced similar degrees of inhibition of DNA synthesis in the untreated abdominal skin.

### Example XXXIII

Topical application of 10% lactic acid in urethane compound 36C (PPG 1000) daily markedly decreased scaling in X-linked ichythosis after 7-10 days of treatment. This clinical effect was obtained in the absence of irritation or allergenicity.

| Effect of Topical 1% Methotrexate in Urethane Compound 35 on Epidermal DNA Synthesis in Hairless Mice | | | | |
|---|---|---|---|---|
| Site | Treatment | Labeling Index | % Inhibition | (p value) |
| Back | 35A | 3.25 ± 1.71 | - | |
| | 1% MTX | 1.77 ± 1.66 | 47 | 0.078 |
| Abdomen (untreated) | 35A | 3.57 ± 1.40 | - | |
| | 1% MTX | 1.85 ± 2.19 | 48 | 0.100 |
| 35A (n = 8) MTX (n = 7) Treatments daily x 10 MTX (Sigma) | | | | |

The above discussion of this invention is directed primarily to preferred embodiments and practices thereof. It will be readily apparent to those skilled in the art that further changes and modifications in the actual implementation of the concepts described herein can readily be made without departing from the spirit and scope of the invention as defined by the following claims.

## Claims (Claims for the following Contracting State(s): AT, BE, CH, DE, FR, GB, IT, LI, LU, NL, SE)

1. A composition for achieving enhanced penetration of a pharmacologically active agent applied dermally or to other epithelial tissue, comprising:
(a) a therapeutically effective amount of a pharmacologically active agent; and
(b) an amount of a permeation enhancer effective to enhance the penetration of the pharmacologically active agent, the enhancer being a compound of formula I 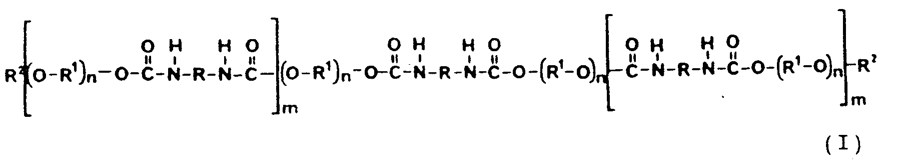
wherein:
R is an alkylene or alkenylene radical containing from one to 20 carbon atoms, or a cycloalkylene or a cycloalkenylene radical containing from 5 to 10 carbon atoms, or a mononuclear or fused ring arylene radical containing from 6 to 10 carbon atoms which R radicals may be unsubstituted or substituted;
R¹ is the same or different alkylene or alkenylene radical;
R² is hydrogen or a methyl or ethyl group;
m is a number in the range of 0 and 6 inclusive; and
n is an integer selected so as to provide the enhancer with a molecular weight of at least 200.

2. A composition according to claim 1 wherein, in the compound of formula I, m is in the range of 0 to 3 inclusive.

3. A composition according to claim 2, wherein m is 0.

4. A composition according to any one of the preceding claims wherein, in the compound of formula I, n is selected so as to give a molecular weight in the range of from 250 to 2200.

5. A composition according to any one of claims 1 to 3, wherein, in the compound of formula I, each n is selected to provide each polyoxyethylene moiety (-O-CH₂-CH₂-)ₙ with a molecular weight of about 500.

6. A composition according to any one of the preceding claims, wherein, in the compound of formula I, R is a mononuclear or fused ring arylene radical which is substituted by one or more C₁-C₆ alkyl, C₁-C₆ alkoxy, C₁-C₆ alkyl-substituted C₁-C₆ alkoxy, amino or nitro groups or halogen atoms.

7. A composition for achieving enhanced penetration of a pharmacologically active agent applied dermally or to other epithelial tissue, comprising:
(a) a therapeutically effective amount of a pharmacologically active agent; and
(b) an amount of a permeation enhancer effective to enhance the penetration of the pharmacologically active agent, the enhancer being a compound of formula IA 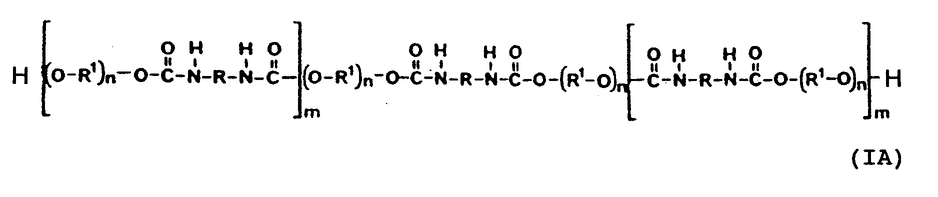
wherein:
R is an alkylene or alkenylene radical containing from one to 20 carbon atoms, or a cycloalkylene or a cycloalkenylene radical containing from 5 to 10 carbon atoms, or a mononuclear or fused ring arylene radical containing from 6 to 10 carbon atoms which R radicals may be unsubstituted or substituted;
R¹ is the same or different alkylene or alkenylene radical;
m is a number in the range of 0 and 6 inclusive; and
n is an integer selected so as to provide the enhancer with a molecular weight of at least 200.

8. A composition according to any one of the preceding claims wherein the pharmacologically active agent is hydrocortisone.

9. A composition according to any one of claims 1 to 7 wherein the pharmacologically active agent is methotrexate.

10. A composition according to any one of the preceding claims, wherein the composition further contains a topical carrier.

11. A composition according to claim 10, wherein the topical carrier comprises water and alcohol.

12. A composition according to claim 11, wherein the topical carrier comprises about 30 wt%, about 45 wt% alcohol, and about 25 wt% of the enhancer.

13. A composition according to claim 11 or 12, wherein the alcohol is selected from isopropanol, ethanol and mixtures thereof.

14. A composition according to any one of the preceding claims, wherein the enhancer is present in an amount of from 5 wt% to 90 wt% of the composition.

15. A composition according to claim 14, wherein the enhancer is present in an amount of from 5 wt% to 25 wt% of the composition.

16. A compound having the formula 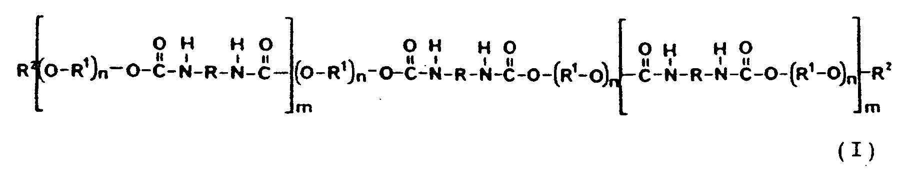 wherein:
R is an alkylene or alkenylene radical containing from up to 20 carbon atoms, or a cycloalkylene or a cycloalkenylene radical containing from 5 to 10 carbon atoms, or a mononuclear or fused ring arylene radical containing from 6 to 10 carbon atoms which R radicals may be unsubstituted or substituted;
R¹ is the same or different alkylene or alkenylene radical;
R² is hydrogen or a methyl or ethyl group;
m is a number in the range of 0 and 6 inclusive; and
n is an integer selected so as to provide the compound with a molecular weight of at least 200 for use in medical treatment by enhancing the penetration of a pharmacologically active agent applied dermally or to other epithelial tissue.

17. A compound according to claim 16, wherein, in the compound of formula I, m is in the range of 0 to 3 inclusive.

18. A compound according to claim 17, wherein m is 0.

19. A compound according to any one of claims 16 to 18 wherein n is selected so as to give a molecular weight in the range of from 250 to 2200.

20. A compound according to any one of claims 16 to 18, wherein, in the compound of formula I, each n is selected to provide each polyoxyethylene moiety (-O-CH₂-CH₂-)ₙ with a molecular weight of about 500.

21. A compound according to any one of claims 16 to 20, wherein, in the compound of formula I, R is a mononuclear or fused ring arylene radical which is substituted by one or more C₁-C₆ alkyl, C₁-C₆ alkoxy, C₁-C₆ alkyl-substituted C₁-C₆ alkoxy, amino or nitro groups or halogen atoms.

22. A compound having the formula 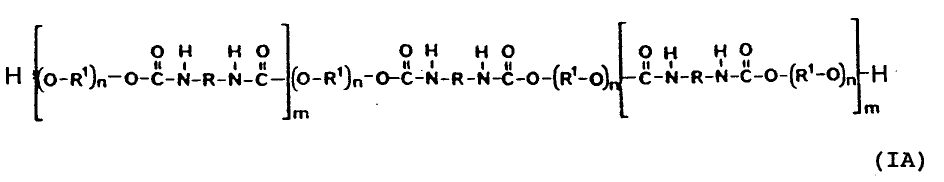 wherein:
R is an alkylene or alkenylene radical containing from one to 20 carbon atoms, or a cycloalkylene or a cycloalkenylene radical containing from 5 to 10 carbon atoms, or a mononuclear or fused ring arylene radical containing from 6 to 10 carbon atom which R radicals may be unsubstituted or substituted;
R¹ is the same or different alkylene or alkenylene radical;
m is a number in the range of 0 and 6 inclusive; and
n is an integer selected so as to provide the compound with a molecular weight of at least 200 for use in medical treatment by enhancing the penetration of a pharmacologically active agent applied dermally or to other epithelial tissue.

23. A compound according to any one of claims 16 to 22 for use in enhancing the penetration of hydrocortisone.

24. A compound according to any one of claims 16 to 22 for use in enhancing the penetration of methotrexate.

## Claims (Claims for the following Contracting State(s): ES, GR)

1. A process for preparing composition for achieving enhanced penetration of a pharmacologically active agent applied dermally or to other epithelial tissue, which process comprises mixing:
(a) a therapeutically effective amount of a pharmacologically active agent; and
(b) an amount of a permeation enhancer effective to enhance the penetration of the pharmacologically active agent, the enhancer being a compound of formula I 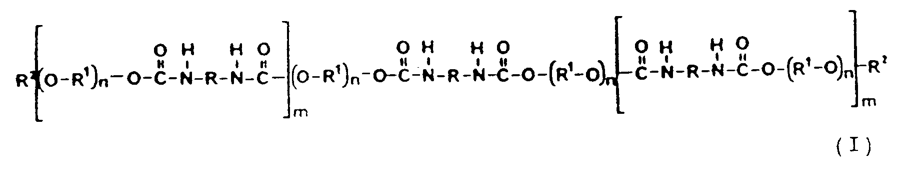
wherein:
R is an alkylene or alkenylene radical containing from one to 20 carbon atoms, or a cycloalkylene or a cycloalkenylene radical containing from 5 to 10 carbon atoms, or a mononuclear or fused ring arylene radical containing from 6 to 10 carbon atoms which R radicals may be unsubstituted or substituted;
R¹ is the same or different alkylene or alkenylene radical;
R² is hydrogen or a methyl or ethyl group;
m is a number in the range of 0 and 6 inclusive; and
n is an integer selected so as to provide the enhancer with a molecular weight of at least 200.

2. A process according to claim 1 wherein, in the compound of formula I used, m is in the range of 0 to 3 inclusive.

3. A process according to claim 2, wherein m is 0.

4. A process according to any one of the preceding claims wherein, in the compound of formula I used, n is selected so as to give a molecular weight in the range of from 250 to 2200.

5. A process according to any one of claims 1 to 3, wherein, in the compound of formula I used, each n is selected to provide each polyoxyethylene moiety (-O-CH₂-CH₂-)ₙ with a molecular weight of about 500.

6. A process according to any one of the preceding claims, wherein, in the compound of formula I used, R is a mononuclear or fused ring arylene radical which is substituted by one or more C₁-C₆ alkyl, C₁-C₆ alkoxy, C₁-C₆ alkyl-substituted C₁-C₆ alkoxy, amino or nitro groups or halogen atoms.

7. A process for achieving enhanced penetration of a pharmacologically active agent applied dermally or to other epithelial tissue, which process comprises mixing:
(a) a therapeutically effective amount of a pharmacologically active agent; and
(b) an amount of a permeation enhancer effective to enhance the penetration of the pharmacologically active agent, the enhancer being a compound of formula IA 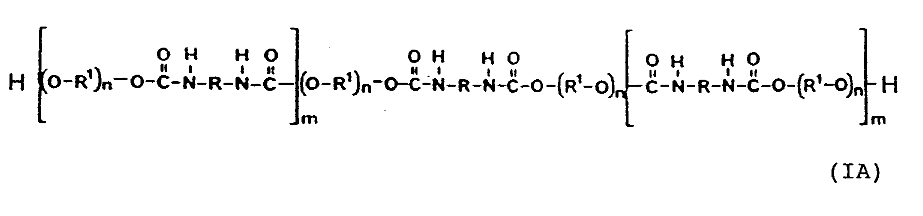
wherein:
R is an alkylene or alkenylene radical containing from one to 20 carbon atoms, or a cycloalkylene or a cycloalkenylene radical containing from 5 to 10 carbon atoms, or a mononuclear or fused ring arylene radical containing from 6 to 10 carbon atoms which R radicals may be unsubstituted or substituted;
R¹ is the same or different alkylene or alkenylene radical;
m is a number in the range of 0 and 6 inclusive; and
n is an integer selected so as to provide the enhancer with a molecular weight of at least 200.

8. A process according to any one of the preceding claims wherein the pharmacologically active agent used is hydrocortisone.

9. A process according to any one of claims 1 to 7 wherein the pharmacologically active agent used is methotrexate.

10. A process according to any one of the preceding claims, wherein there is also used a topical carrier.

11. A process according to claim 10, wherein the topical carrier used comprises water and alcohol.

12. A process according to claim 11, wherein the topical carrier comprises about 30 wt%, about 45 wt% alcohol, and about 25 wt% of the enhancer.

13. A process according to claim 11 or 12, wherein the alcohol used is selected from isopropanol, ethanol and mixtures thereof.

14. A process according to any one of the preceding claims, wherein the enhancer is used in an amount of from 5 wt% to 90 wt% of the composition.

15. A process according to claim 14, wherein the enhancer is used in an amount of from 5 wt% to 25 wt% of the composition.

## Patentansprüche (Patentansprüche für folgende(n) Vertragsstaat(en): AT, BE, CH, DE, FR, GB, IT, LI, LU, NL, SE)

1. Zusammensetzung, um ein verbessertes Eindringen eines pharmakologisch wirksamen Mittels, welches auf die Haut oder auf anderes Epithelgewebe aufgetragen wurde, zu erzielen, umfassend
(a) eine therapeutisch wirksame Menge eines pharmakologisch wirksamen Mittels; und
(b) eine Menge eines Permeationsverstärkers, welche wirksam ist, das Eindringen des pharmakologisch wirksamen Mittels zu verstärken,
wobei der Verstärker eine Verbindung der Formel (I) 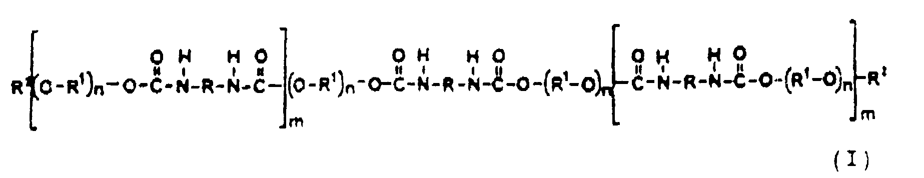 ist, in welcher:
R ein Alkylen- oder Alkenylenrest mit 1 bis 20 Kohlenstoffatomen oder ein Cycloalkylen- oder ein Cycloalkenylenrest mit 5 bis 10 Kohlenstoffatomen oder ein einkerniger oder in eine Ringstruktur eingebundener Arylenrest mit 6 bis 10 Kohlenstoffatomen ist, wobei die Reste R nicht substituiert oder substituiert sein können,
R¹ derselbe oder ein andersartiger Alkylen- oder Alkenylenrest ist,
R² Wasserstoff oder eine Methyl- oder Ethylgruppe ist,
m eine Zahl zwischen 0 und einschließlich 6 ist, und
n eine ganze Zahl ist, welche so ausgewählt wird, daß der Verstärker mit einem Molekulargewicht von mindestens 200 bereitgestellt wird.

2. Zusammensetzung nach Anspruch 1,
wobei in der Verbindung der Formel I m zwischen 0 und einschließlich 3 ist.

3. Zusammensetzung nach Anspruch 2,
in welcher m 0 ist.

4. Zusammensetzung nach irgendeinem der vorangegangenen Ansprüche,
wobei in der Verbindung der Formel I n so ausgewählt wird, daß ein Molekulargewicht zwischen 250 und 2200 erhalten wird.

5. Zusammensetzung nach irgendeinem der Ansprüche 1 bis 3,
wobei in der Verbindung der Formel I jedes n so ausgewählt wird, daß jede Polyoxyethylen-Gruppierung (-O-CH₂-CH₂-)ₙ mit einem Molekulargewicht von ungefähr 500 vorgesehen wird.

6. Zusammensetzung nach irgendeinem der vorangegangenen Ansprüche,
wobei in der Verbindung der Formel I R ein einkerniger oder in eine Ringstruktur eingebundener Arylenrest ist, der mit einer oder mehreren C₁-C₆-Alkyl-, C₁-C₆-Alkoxy-, C₁-C₆-alkylsubstituierten C₁-C₆-Alkoxy-, Amino- oder Nitrogruppen oder Halogenatomen substituiert ist.

7. Zusammensetzung, um ein verbessertes Eindringen eines pharmakologisch wirksamen Mittels, welches auf die Haut oder anderes Epithelgewebe aufgetragen wurde, zu erzielen,
umfassend
(a) eine therapeutisch wirksame Menge eines pharmakologisch wirksamen Mittels; und
(b) eine Menge eines Permeationsverstärkers, welche wirksam ist, das Eindringen des pharmakologisch wirksamen Mittels zu verstärken,
wobei der Verstärker eine Verbindung der Formel (IA) 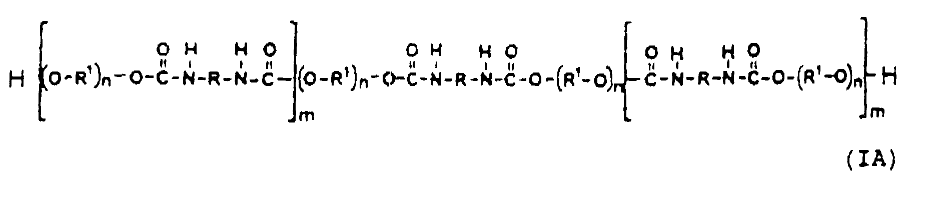 ist, in welcher:
R ein Alkylen- oder Alkenylenrest mit 1 bis 20 Kohlenstoffatomen oder ein Cycloalkylen- oder ein Cycloalkenylenrest mit 5 bis 10 Kohlenstoffatomen oder ein einkerniger oder in eine Ringstruktur eingebundener Arylenrest mit 6 bis 10 Kohlenstoffatomen ist, wobei die Reste R nicht substituiert oder substituiert sein können,
R¹ derselbe oder ein andersartiger Alkylen- oder Alkenylenrest ist,
m eine Zahl Zwischen 0 und einschließlich 6 ist, und
n eine ganze Zahl ist, welche so ausgewählt wird, daß der Verstärker mit einem Molekulargewicht von mindestens 200 bereitgestellt wird.

8. Zusammensetzung nach irgendeinem der vorangegangenen Ansprüche,
in welcher das pharmakologisch wirksame Mittel Hydrocortison ist.

9. Zusammensetzung nach irgendeinem der Ansprüche 1 bis 7,
in welcher das pharmakologisch wirksame Mittel Methotrexat ist.

10. Zusammensetzung nach irgendeinem der vorangegangenen Ansprüche,
in welcher die Zusammensetzung ferner einen topischen Träger umfaßt.

11. Zusammensetzung nach Anspruch 10,
in welcher der topische Träger Wasser und Alkohol umfaßt.

12. Zusammensetzung nach Anspruch 11,
in welcher der topische Träger ungefähr 30 Gew.-% Wasser, ungefähr 45 Gew.-% Alkohol und ungefähr 25 Gew.-% Verstärker umfaßt.

13. Zusammensetzung nach Anspruch 11 oder 12,
in welcher der Alkohol ausgewählt ist aus Isopropanol, Ethanol und Mischungen derselben.

14. Zusammensetzung nach irgendeinem der vorangegangenen Ansprüche,
in welcher der Verstärker in einer Menge von 5 Gew.-% bis 90 Gew.-% der Zusammensetzung vorliegt.

15. Zusammensetzung nach Anspruch 14,
in welcher der Verstärker in einer Menge von 5 Gew.-% bis 25 Gew.-% der Zusammensetzung vorliegt.

16. Verbindung der Formel 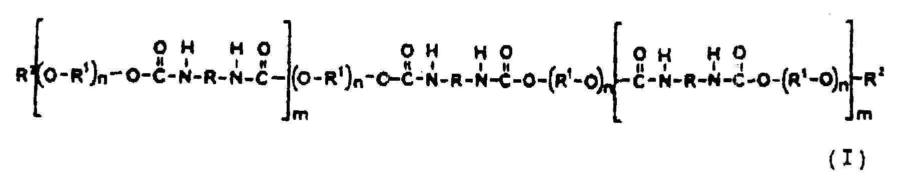 in welcher:
R ein Alkylen- oder Alkenylenrest mit 1 bis 20 Kohlenstoffatomen oder ein Cycloalkylen- oder ein Cycloalkenylenrest mit 5 bis 10 Kohlenstoffatomen oder ein einkerniger oder in eine Ringstruktur eingebundener Arylenrest mit 6 bis 10 Kohlenstoffatomen ist, wobei die Reste R nicht substituiert oder substituiert sein können,
R¹ derselbe oder ein andersartiger Alkylen- oder Alkenylenrest ist,
R² Wasserstoff oder eine Methyl- oder Ethylgruppe ist,
m eine Zahl zwischen 0 und einschließlich 6 ist, und
n eine ganze Zahl ist, welche so ausgewählt wird, daß die Verbindung mit einem Molekulargewicht von mindestens 200 bereitgestellt wird zur Verwendung in einer medizinischen Behandlung durch Verstärkung des Eindringens eines pharmakologisch wirksamen Mittels, welches auf die Haut oder auf anderes Epithelgewebe aufgetragen wurde.

17. Verbindung nach Anspruch 16,
wobei in der Verbindung der Formel I m zwischen 0 und einschließlich 3 ist.

18. Verbindung nach Anspruch 17,
in welcher m 0 ist.

19. Verbindung nach irgendeinem der Ansprüche 16 bis 18,
in welcher n so ausgewählt wird, daß sich ein Molekulargewicht zwischen 250 und 2200 ergibt.

20. Verbindung nach irgendeinem der Ansprüche 16 bis 18,
wobei in der Verbindung der Formel I jedes n so ausgewählt wird, daß jede Polyoxyethylen-Gruppierung (-O-CH₂-CH₂-)ₙ mit einem Molekulargewicht von ungefähr 500 vorgesehen wird.

21. Verbindung nach irgendeinem der Ansprüche 16 bis 20,
wobei in der Verbindung der Formel I R ein einkerniger oder in eine Ringstruktur eingebundener Arylenrest ist, der mit einer oder mehreren C₁-C₆-Alkyl-, C₁-C₆-Alkoxy-, C₁-C₆-alkylsubstituierten C₁-C₆-Alkoxy-, Amino- oder Nitrogruppen oder Halogenatomen substituiert ist.

22. Verbindung mit der Formel 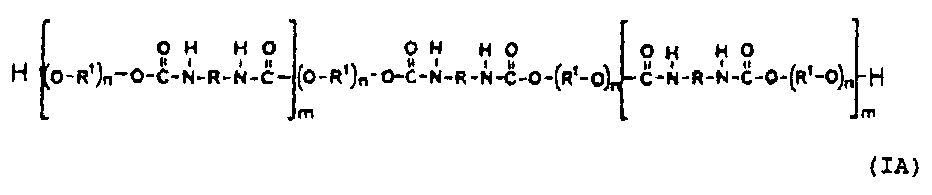 in welcher:
R ein Alkylen- oder Alkenylenrest mit 1 bis 20 Kohlenstoffatomen oder ein Cycloalkylen- oder ein Cycloalkenylenrest mit 5 bis 10 Kohlenstoffatomen oder ein einkerniger oder in eine Ringstruktur eingebundener Arylenrest mit 6 bis 10 Kohlenstoffatomen ist, wobei die Reste R nicht substituiert oder substituiert sein können,
R¹ derselbe oder ein andersartiger Alkylen- oder Alkenylenrest ist,
m eine Zahl zwischen 0 und einschließlich 6 ist, und
n eine ganze Zahl ist, welche so ausgewählt wird, daß die Verbindung mit einem Molekulargewicht von mindestens 200 bereitgestellt wird zur Verwendung in einer medizinischen Behandlung durch eine Verstärkung des Eindringens eines pharmakologisch wirksamen Mittels, welches auf die Haut oder auf anderes Epithelgewebe aufgetragen wurde.

23. Verbindung nach irgendeinem der Ansprüche 16 bis 22 zur Verwendung zur Verstärkung des Eindringens von Hydrocortison.

24. Verbindung nach irgendeinem der Ansprüche 16 bis 22 zur Verwendung zur Verstärkung des Eindringens von Methotrexat.

## Patentansprüche (Patentansprüche für folgende(n) Vertragsstaat(en): ES, GR)

1. Verfahren zur Herstellung einer Zusammensetzung, um ein verbessertes Eindringen eines pharmakologisch wirksamen Mittels, welches auf die Haut oder auf anderes Epithelgewebe aufgetragen wurde, zu erzielen, wobei das Verfahren ein Mischen umfaßt von:
(a) einer therapeutisch wirksamen Menge eines pharmakologisch wirksamen Mittels; und
(b) einer Menge eines Permeationsverstärkers, welche wirksam ist, das Eindringen des pharmakologisch wirksamen Mittels zu verstärken,
wobei der Verstärker eine Verbindung der Formel (I) 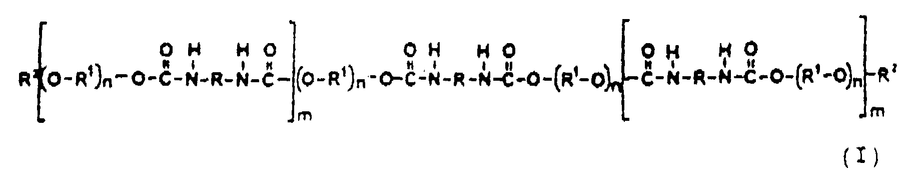 ist, in welcher:
R ein Alkylen- oder Alkenylenrest mit 1 bis 20 Kohlenstoffatomen oder ein Cycloalkylen- oder ein Cycloalkenylenrest mit 5 bis 10 Kohlenstoffatomen oder ein einkerniger oder in eine Ringstruktur eingebundener Arylenrest mit 6 bis 10 Kohlenstoffatomen ist, wobei die Reste R nicht substituiert oder substituiert sein können,
R¹ derselbe oder ein andersartiger Alkylen- oder Alkenylenrest ist,
R² Wasserstoff oder eine Methyl- oder Ethylgruppe ist,
m eine Zahl zwischen 0 und einschließlich 6 ist, und
n eine ganze Zahl ist, welche so ausgewählt wird, daß der Verstärker mit einem Molekulargewicht von mindestens 200 bereitgestellt wird.

2. Verfahren nach Anspruch 1,
wobei in der verwendeten Verbindung der Formel I m zwischen 0 und einschließlich 3 ist.

3. Verfahren nach Anspruch 2,
in welcher m 0 ist.

4. Verfahren nach irgendeinem der vorangegangenen Ansprüche,
wobei in der verwendeten Verbindung der Formel I n so ausgewählt wird, daß ein Molekulargewicht zwischen 250 und 2200 erhalten wird.

5. Verfahren nach irgendeinem der Ansprüche 1 bis 3,
wobei in der verwendeten Verbindung der Formel I jedes n so ausgewählt wird, daß jede Polyoxyethylen-Gruppierung (-O-CH₂-CH₂-)ₙ mit einem Molekulargewicht von ungefähr 500 vorgesehen wird.

6. Verfahren nach irgendeinem der vorangegangenen Ansprüche,
wobei in der verwendeten Verbindung der Formel I R ein einkerniger oder in eine Ringstruktur eingebundener Arylenrest ist, der mit einer oder mehreren C₁-C₆-Alkyl-, C₁-C₆-Alkoxy-, C₁-C₆-alkylsubstituierten C₁-C₆-Alkoxy-, Amino- oder Nitrogruppen oder Halogenatomen substituiert ist.

7. Verfahren, um ein verbessertes Eindringen eines pharmakologisch wirksamen Mittels, welches auf die Haut oder auf anderes Epithelgewebe aufgetragen wurde, zu erzielen,
wobei das Verfahren ein Mischen umfaßt von:
(a) einer therapeutisch wirksamen Menge eines pharmakologisch wirksamen Mittels; und
(b) einer Menge eines Permeationsverstärkers, welche wirksam ist, das Eindringen des pharmakologisch wirksamen Mittels zu verstärken,
wobei der Verstärker eine Verbindung der Formel (IA) 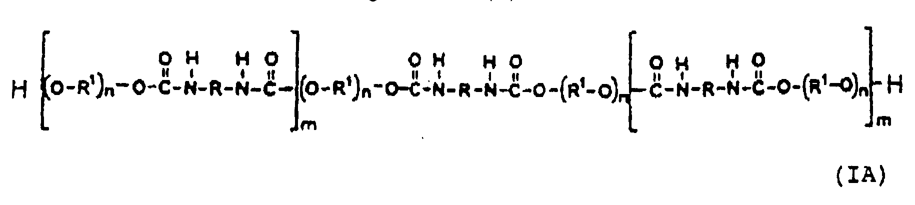 ist, in welcher:
R ein Alkylen- oder Alkenylenrest mit 1 bis 20 Kohlenstoffatomen oder ein Cycloalkylen- oder ein Cycloalkenylenrest mit 5 bis 10 Kohlenstoffatomen oder ein einkerniger oder in eine Ringstruktur eingebundener Arylenrest mit 6 bis 10 Kohlenstoffatomen ist, wobei die Reste R nicht substituiert oder substituiert sein können,
R¹ derselbe oder ein andersartiger Alkylen- oder Alkenylenrest ist,
m eine Zahl zwischen 0 und einschließlich 6 ist, und
n eine ganze Zahl ist, welche so ausgewählt wird, daß der Verstärker mit einem Molekulargewicht von mindestens 200 bereitgestellt wird.

8. Verfahren nach irgendeinem der vorangegangenen Ansprüche,
in welchem das verwendete pharmakologisch wirksame Mittel Hydrocortison ist.

9. Verfahren nach irgendeinem der Ansprüche 1 bis 7,
in welchem das verwendete pharmakologisch wirksame Mittel Methotrexat ist.

10. Verfahren nach irgendeinem der vorangegangenen Ansprüche,
in welchem ferner ein topischer Träger verwendet wird.

11. Verfahren nach Anspruch 10,
in welchem der verwendete topische Träger Wasser und Alkohol umfaßt.

12. Verfahren nach Anspruch 11,
in welchem der topische Träger ungefähr 30 Gew.-% Wasser, ungefähr 45 Gew.-% Alkohol und ungefähr 25 Gew.-% Verstärker umfaßt.

13. Verfahren nach Anspruch 11 oder 12,
in welchem der verwendete Alkohol ausgewählt wird aus Isopropanol, Ethanol und Mischungen derselben.

14. Verfahren nach irgendeinem der vorangegangenen Ansprüche,
in welchem der Verstärker in einer Menge von 5 Gew.-% bis 90 Gew.-% der Zusammensetzung verwendet wird.

15. Verfahren nach Anspruch 14,
in welchem der Verstärker in einer Menge von 5 Gew.-% bis 25 Gew.-% der Zusammensetzung verwendet wird.

## Revendications (Revendications pour l'(les) Etat(s) contractant(s) suivant(s): AT, BE, CH, DE, FR, GB, IT, LI, LU, NL, SE)

1. Composition pour améliorer la pénétration d'un principe actif pharmacologique appliqué sur la peau ou sur d'autres tissus épithéliaux, comprenant :
(a) une quantité thérapeutiquement efficace d'un principe actif pharmacologique, et
(b) une quantité d'un agent favorisant la perméation suffisante pour augmenter la pénétration du principe actif pharmacologique, l'agent favorisant la perméation étant un composé de formule I 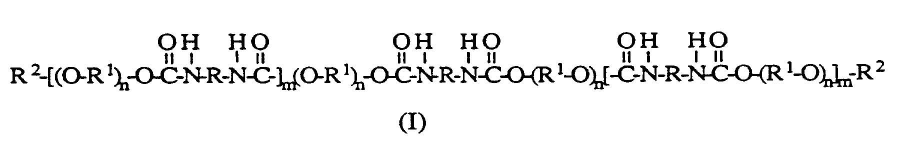
dans laquelle
R représente un groupe alkylène ou alcénylène comportant de 1 à 20 atomes de carbone, ou un radical cycloalkylène ou cycloalcénylène comportant de 5 à 10 atomes de carbone, ou un radical arylène à noyau unique ou comportant des cycles condensés contenant de 6 à 10 atomes de carbone, ces radicaux R pouvant être substitués ou non
R¹ représente un radical alkylène ou alcénylène identique ou différent,
R² représente un atome d'hydrogène ou un groupe méthyle ou éthyle,
m est un nombre compris entre 0 et 6 inclus,
n est un nombre entier choisi de manière à ce que l'agent favorisant la perméation ait une masse moléculaire au moins égale à 200.

2. Composition conforme à la revendication 1 dans laquelle, dans le composé de formule I, m est compris entre 0 et 3 inclus.

3. Composition conforme à la revendication 2 dans laquelle m est égal à 0.

4. Composition conforme à une quelconque des revendications précédentes dans laquelle, dans le composé de formule I, n est choisi ne manière à obtenir une masse moléculaire comprise entre 250 et 2200.

5. Composition conforme à une des revendications 1 à 3, dans laquelle, dans le composé de formule I, chaque n est choisi de manière à ce que chaque séquence poly(éthylène oxyde) (-O-CH₂-CH₂)ₙ ait une masse moléculaire d'environ 500.

6. Composition conforme à une quelconque des revendications précédentes dans laquelle, dans le composé de formule I, R représente un radical arylène à noyau unique ou constitué de cycles condensés, substitué par un ou plusieurs groupes alkyle en C₁-C₆, alcoxy en C₁-C₆, alcoxy en C₁-C₆ substitué par un alkyle en C₁-C₆, amino ou nitro ou des atomes d'halogène.

7. Composition pour améliorer la pénétration d'un principe actif pharmacologique appliqué sur la peau ou sur d'autres tissus épithéliaux, comprenant
(a) une quantité thérapeutiquement efficace d'un principe actif pharmacologique, et
(b) une quantité d'un agent favorisant la perméation suffisante pour augmenter la pénétration du principe actif pharmacologique, l'agent favorisant la perméation étant un composé de formule IA : 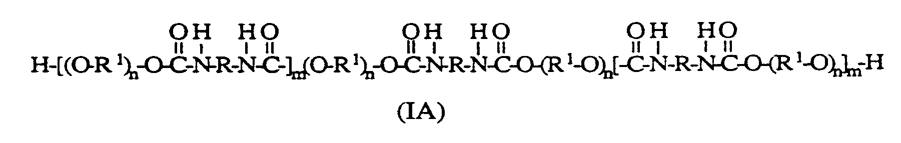
dans laquelle
R représente un groupe alkylène ou alcénylène comportant de 1 à 20 atomes de carbone, ou un radical cycloalkylène ou cycloalcénylène comportant de 5 à 10 atomes de carbone, ou un radical arylène à noyau unique ou comportant des cycles condensés contenant de 6 à 10 atomes de carbone, ces radicaux R pouvant être substitués ou non
R¹ représente un radical alkylène ou alcénylène identique ou différent,
m est un nombre entier compris entre 0 et 6 inclus,
n est un nombre entier choisi de manière à ce que l'agent favorisant la perméation ait une masse moléculaire au moins égale à 200.

8. Composition conforme à une quelconque des revendications précédentes dans laquelle le principe actif pharmacologique est l'hydrocortisone.

9. Composition conforme à une quelconque des revendications 1 à 7 dans laquelle le principe actif pharmacologique est le méthotrexate.

10. Composition conforme à une quelconque des revendications précédentes dans laquelle la composition contient en outre un véhicule pour applications locales.

11. Composition conforme à la revendication 10 dans laquelle le véhicule pour applications locales contient de l'eau et de l'alcool.

12. Composition conforme à la revendication 11 dans laquelle le véhicule pour applications locales contient environ 30 % en poids d'eau, environ 45 % en poids d'alcool et environ 25 % de l'agent favorisant la pénétration.

13. Composition conforme à la revendication 11 ou 12 dans laquelle l'alcool est choisi parmi l'isopropanol, l'éthanol et des mélanges de ceux-ci.

14. Composition conforme à une quelconque des revendications précédentes dans laquelle l'agent favorisant la perméation est présent à raison de 5 à 90 % en poids de la composition.

15. Composition conforme à une quelconque des revendications précédentes dans laquelle l'agent favorisant la perméation est présent à raison de 5 à 25 % en poids de la composition.

16. Composé ayant la formule I 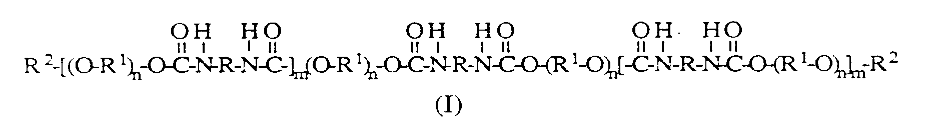 dans laquelle
R représente un groupe alkylène ou alcénylène comportant de 1 à 20 atomes de carbone, ou un radical cycloalkylène ou cycloalcénylène comportant de 5 à 10 atomes de carbone, ou un radical arylène à noyau unique ou constitué de cycles condensés comportant de 6 à 10 atomes de carbone, ces radicaux R pouvant être substitués ou non
R¹ représente un radical alkylène ou alcényle identique ou différent,
R² représente un atome d'hydrogène ou un groupe méthyle ou éthyle,
m est un nombre compris entre 0 et 6 inclus,
n est un nombre entier choisi de manière à ce que l'agent favorisant la perméation ait une masse moléculaire au moins égale à 200,
utilisé pour améliorer la pénétration d'un principe actif pharmacologique appliqué sur la peau ou sur d'autres tissus épithéliaux lors d'un traitement médical.

17. Composé conforme à la revendication 16 dans lequel, dans le composé de formule I, m est compris entre 0 et 3 inclus.

18. Composé conforme à la revendication 17 dans lequel m est égal à 0.

19. Composé conforme à une des revendications 16 à 18 dans lequel n est choisi de manière à obtenir une masse moléculaire comprise entre 250 et 2200.

20. Composé conforme à une quelconque des revendications 16 à 18 dans lequel, dans le composé de formule I, chaque n est choisi de manière à ce que chaque séquence poly(éthylène oxyde) (-O-CH₂-CH₂)ₙ ait une masse moléculaire d'environ 500.

21. Composé conforme à une quelconque des revendications 16 à 20 dans lequel, dans le composé de formule I, R représente un radical arylène à noyau unique ou constitué de cycles condensés, substitué par un ou plusieurs groupes alkyle en C₁-C₆, alcoxy en C₁-C₆, alcoxy en C₁-C₆ substitué par un alkyle en C₁-C₆, amino ou nitro ou par des atomes d'halogène.

22. Composé de formule 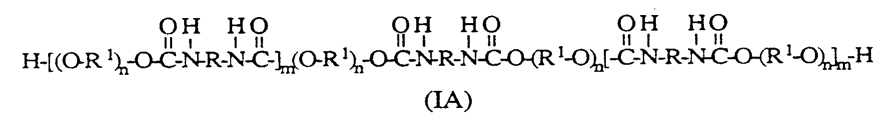 dans laquelle
R représente un groupe alkylène ou alcénylène comportant de 1 à 20 atomes de carbone, ou un radical cycloalkylène ou cycloalcénylène comportant de 5 à 10 atomes de carbone, ou un radical arylène à noyau unique ou constitué de cycles condensés comportant de 6 à 10 atomes de carbone, ces radicaux R pouvant être substitués ou non
R¹ représente un radical alkylène ou alcényle identique ou différent,
m est un nombre compris entre 0 et 6 inclus,
n est un nombre entier choisi de manière à ce que l'agent favorisant la perméation ait une masse moléculaire au moins égale à 200
utilisé pour améliorer la pénétration d'un principe actif pharmacologique appliqué sur la peau ou sur d'autres tissus épithéliaux lors d'un traitement médical.

23. Composé conforme à une quelconque des revendications 16 à 22 utilisé pour augmenter la pénétration de l'hydrocortisone.

24. Composé conforme à une quelconque des revendications 16 à 22 utilisé pour augmenter la pénétration du méthotrexate.

## Revendications (Revendications pour l'(les) Etat(s) contractant(s) suivant(s): ES, GR)

1. Procédé de préparation d'une composition pour améliorer la pénétration d'un principe actif pharmacologique appliqué sur la peau ou sur d'autres tissus épithéliaux, comprenant :
(a) une quantité thérapeutiquement efficace d'un principe actif pharmacologique, et
(b) une quantité d'un agent favorisant la perméation suffisante pour augmenter la pénétration du principe actif pharmacologique, l'agent favorisant la perméation étant un composé de formule I 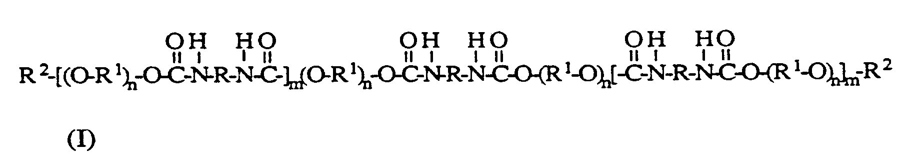
dans laquelle
R représente un groupe alkylène ou alcénylène comportant de 1 à 20 atomes de carbone, ou un radical cycloalkylène ou cycloalcénylène comportant de 5 à 10 atomes de carbone, ou un radical arylène à noyau unique ou comportant des cycles condensés contenant de 6 à 10 atomes de carbone, ces radicaux R pouvant être substitués ou non
R¹ représente un radical alkylène ou alcénylène identique ou différent,
R² représente un atome d'hydrogène ou un groupe méthyle ou éthyle,
m est un nombre compris entre 0 et 6 inclus,
n est un nombre entier choisi de manière à ce que l'agent favorisant la perméation ait une masse moléculaire au moins égale à 200.

2. Procédé conforme à la revendication 1 dans lequel, dans le composé de formule I utilisé, m est compris entre 0 et 3 inclus.

3. Procédé conforme à la revendication 2 dans lequel m est égal à 0.

4. Procédé conforme à une quelconque des revendications précédentes dans lequel, dans le composé de formule I utilisé, n est choisi ne manière à obtenir une masse moléculaire comprise entre 250 et 2200.

5. Procédé conforme à une des revendications 1 à 3, dans lequel, dans le composé de formule I utilisé, chaque n est choisi de manière à ce que chaque séquence poly(éthylène oxyde) (-O-CH₂-CH₂)ₙ ait une masse moléculaire d'environ 500.

6. Procédé conforme à une quelconque des revendications précédentes dans lequel, dans le composé de formule I utilisé, R représente un radical arylène à noyau unique ou constitué de cycles condensés, substitué par un ou plusieurs groupes alkyle en C₁-C₆, alcoxy en C₁-C₆, alcoxy en C₁-C₆ substitué par un alkyle en C₁-C₆, amino ou nitro ou des atomes d'halogène.

7. Procédé pour améliorer la pénétration d'un principe actif pharmacologique appliqué sur la peau ou sur d'autres tissus épithéliaux, comprenant
(a) une quantité thérapeutiquement efficace d'un principe actif pharmacologique, et
(b) une quantité d'un agent favorisant la perméation suffisante pour augmenter la pénétration du principe actif pharmacologique, l'agent favori sant la perméation étant un composé de formule IA : 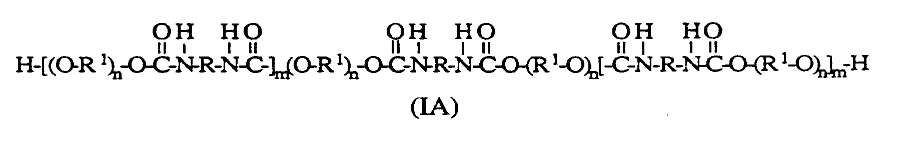
dans laquelle
R représente un groupe alkylène ou alcénylène comportant de 1 à 20 atomes de carbone, ou un radical cycloalkylène ou cycloalcénylène comportant de 5 à 10 atomes de carbone, ou un radical arylène à noyau unique ou comportant des cycles condensés contenant de 6 à 10 atomes de carbone, ces radicaux R pouvant être substitués ou non
R¹ représente un radical alkylène ou alcénylène identique ou différent,
m est un nombre entier compris entre 0 et 6 inclus, et
n est un nombre entier choisi de manière à ce que l'agent favorisant la perméation ait une masse moléculaire au moins égale à 200.

8. Procédé conforme à une quelconque des revendications précédentes dans lequel le principe actif pharmacologique utilisé est l'hydrocortisone.

9. Procédé conforme à une quelconque des revendications 1 à 7 dans lequel le principe actif pharmacologique utilisé est le méthotrexate.

10. Procédé conforme à une quelconque des revendications précédentes dans lequel on utilise en outre un véhicule pour applications locales.

11. Procédé conforme à la revendication 10 dans lequel le véhicule pour applications locales utilisé contient de l'eau et de l'alcool.

12. Procédé conforme à la revendication 11 dans lequel le véhicule pour applications locales utilisé contient environ 30 % en poids d'eau, environ 45 % en poids d'alcool et environ 25 % de l'agent favorisant la pénétration.

13. Procédé conforme à la revendication 11 ou 12 dans lequel l'alcool est choisi parmi l'isopropanol, l'éthanol et des mélanges de ceux-ci.

14. Procédé conforme à une quelconque des revendications précédentes dans lequel l'agent favorisant la perméation est utilisé à raison de 5 à 90 % en poids de la composition.

15. Procédé conforme à une quelconque des revendications précédentes dans lequel l'agent favorisant la perméation est utilisé à raison de 5 à 25 % en poids de la composition.
